# EUROPEAN PATENT APPLICATION

(11) **EP 1 201 769 A2**
(43) Date of publication of application: **02.05.2002**
(21) Application number: 01308700.2
(22) Date of filing: 12.10.2001
(51) Int. Cl.: C12Q 1/68

(54) **Gene expression monitoring using universal arrays**

(30) Priority: 12.10.2000 US 240395 P; 21.12.2000 US 747004
(71) Applicant: Affymetrix, Inc., Santa Clara, CA 95051 (US)
(72) Inventor: Hu, Jing-Shan, Mountain View, California 94040 (US); Kaplan, Paul, Campbell, California 95008 (US); Patil, Nila, Woodside, California 94062 (US)
(74) Representative: Chapman, Paul William

(57) **Abstract**

Methods are provided for monitoring a large number of genes using cipher probes. In preferred embodiments, the cipher probes are immobilized on a substrate to form a universal array that is suitable for monitoring the expression of almost any genes. Mediator probes are used in some embodiments to hybridize with the cipher probes and nucleic acids derived from transcripts of genes.

## Description

This application is related to and claims the priority of U.S. Provisional Application Number 60/240,395, filed on October 12, 2000, which is incorporated herein in its entirety by reference for all purposes.

### TECHNICAL FIELD

The present invention is in the field of genetic analysis for medical diagnosis, genetic variation research, or genetic engineering. More specifically, the present invention is in the field of nucleic acid analysis.

### BACKGROUND

Many cellular events and processes are characterized by altered expression levels of one or more genes. Differences in gene expression correlate with many physiological processes such as cell cycle progression, cell differentiation and cell death. Changes in gene expression patterns also correlate with changes in disease or pharmacological state. For example, the lack of sufficient expression of functional tumor suppressor genes and/or the over expression of oncogene/protooncogenes could lead to tumorgenesis (Marshall, Cell, 64: 313-326 (1991); Weinberg, Science, 254: 1138-1146 (1991), incorporated herein by reference in their entireties for all purposes). Thus, changes in the expression levels of particular genes (e.g. oncogenes or tumor suppressors) serve as signposts for different physiological, pharmacological and disease states.

Recently, massive parallel gene expression monitoring methods have been developed to monitor the expression of a large number of genes using nucleic acid array technology which was described in detail in, for example, U.S. Patent Number 5,871,928; de Saizieu, *et al*., 1998, Bacteria Transcript Imaging by Hybridization of total RNA to Oligonucleotide Arrays, NATURE BIOTECHNOLOGY, 16:45-48; Wodicka *et al.*, 1997, Genome-wide Expression Monitoring in *Saccharomyces cerevisiae*, NATURE BIOTECHNOLOGY 15:1359-1367; Lockhart *et al.*, 1996, Expression Monitoring by Hybridization to High Density Oligonucleotide Arrays. NATURE BIOTECHNOLOGY 14:1675-1680; Lander, 1999, Array of Hope, NATURE-GENETICS, 21(suppl.), at 3, all incorporated herein by reference in their entireties for all purposes.

However, there is still great need in the art for additional methods for monitoring the expression of a large number of genes.

### SUMMARY OF THE INVENTION

In one aspect of the invention, methods are provided to use a universal gene expression array with selected cipher probe sequences and mediator probes to monitor expression of any genes of known sequence without having to synthesize a new specific array or obtain the cDNA clones to spot a cDNA array.

A cipher probe is a nucleic acid probe that contains unique sequence. A cipher probe is also referred to as a tag probe (referencing to its ability to bind to a complementary tag sequence) or a capture probe. In preferred embodiments, the methods of the invention are useful for monitoring the expression of a large number of genes indirectly using a universal oligonucleotide array containing the unique sequence ciphers with mediator oligonucleotides. Therefore, generally, the cipher sequences on the array are pre-selected for not containing sequences identical or significantly similar to all known sequences or sequences of genes to be monitored for expression.

The mediators are nucleic acids that bind a complementary cipher probe and a target nucleic acid. In some embodiments, the 3' portion of a mediator oligonucleotide is designed to hybridize to a selected region of the mRNA, cDNA, or cRNA, while its 5' portion is complementary to a specific cipher sequence on the universal array. The gene-specific hybridization of labeled nucleic acid samples to this array happen in the presence of the mediator oligonucleotides and target DNA/RNA, resulting in formation of a ternary complex between the target, mediator oligonucleotide, and cipher oligonucleotide attached to the solid surface. This approach enables monitoring mRNA expression of *any* genes of known sequence, including but not limited to alternatively spliced variants and variants at nucleotides level (*e.g.* SNP), without having to synthesize a new array or to obtain the cDNA clones to spot a cDNA array for the above purpose

In some embodiments for detecting a plurality of nucleic acid targets in a sample. The methods include hybridizing the sample with a plurality of mediator nucleic acids and a plurality of cipher probes immobilized on a substrate, where each of the mediator nucleic acids has a first subsequence that is complementary with one of the nucleic acid targets and a second subsequence that is complementary with one of the cipher probes; and detecting the nucleic acid targets based upon the hybridization pattern.

In some embodiments, the mediator nucleic acids and cipher probes are oligonucleotides. Preferably, the cipher probes do not substantially hybridize with the nucleic acid targets or any nucleic acid in the sample. The cipher probes are at least 15, preferably 20 bases in length. The probes may be immobilized on a substrate, preferably at density of at least 400 or 1000 probes per cm²,

In some embodiments, the mediator oligonucleotides should be at least 15 bases in length, preferably at least 20 or 40 bases in length.

The methods may include quantifying the binding of the nucleic acid targets to the cipher probes through the mediator probes. The sample may be a mRNA sample. Alternatively, the sample may be a cRNA sample derived from a cDNA sample through in vitro transcription. The nucleic acids in the sample may be amplified from a biological sample by an in vivo or an in vitro method.

The target nucleic acids may be labeled with any suitable label.

The cipher probes are synthesized in the 5'-3' direction on the substrate, preferably by photo-directed synthesis. Alternatively, the cipher probes are synthesized in the 3'-5' direction on the substrate, preferably using photo-directed synthesis.

The nucleic acids may be determined using multiple mediators and cipher probes. In preferred embodiments, each of the nucleic acid targets is measured with at least 3, 5, or 10 mediator oligonucleotides and at least 3, 5, or 10 cipher probes.

### BRIEF DESCRIPTION OF THE DRAWINGS

The accompanying drawings, which are incorporated in and form a part of this specification, illustrate embodiments of the invention and, together with the description, serve to explain the principles of the invention:
FIGURE 1 is a schematic illustrating one embodiment of the invention.
FIGURE 2 is another schematic illustrating another embodiment of the invention.
FIGURES 3a and 3b show shows relationship between concentration of the mediator oligos for each oligo and intensity values.
FIGURE 4 shows relationship between cRNA and intensity values.

### DETAILED DESCRIPTION

Reference will now be made in detail to the preferred embodiments of the invention. While the invention will be described in conjunction with the preferred embodiments, it will be understood that they are not intended to limit the invention to these embodiments. On the contrary, the invention is intended to cover alternatives, modifications and equivalents, which may be included within the spirit and scope of the invention.

### I. GENERAL

The present invention relies on many patents, applications and other references for certain details well known to those of the art. Therefore, when a patent, application, or other reference is cited or repeated below, it should be understood that it is incorporated by reference in its entirety for all purposes as well as for the proposition that is recited.

As used in the specification and claims, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention are presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques of organic chemistry, polymer technology, molecular biology (including recombinant techniques), cell biology, biochemistry, and immunology, which are within the skill of the art. Such conventional techniques include polymer array synthesis, hybridization, ligation, detection of hybridization using a label. Such conventional techniques can be found in standard laboratory manuals such as *Genome Analysis: A Laboratory Manual Series (Vols. I-IV), Using Antibodies: A Laboratory Manual, Cells: A Laboratory Manual, PCR Primer: A Laboratory Manual,* and *Molecular Cloning: A Laboratory Manual* (all from Cold Spring Harbor Laboratory Press), all of which are herein incorporated in their entirety by reference for all purposes.

Additional methods and techniques applicable to array synthesis have been described in U.S. Patents Nos. 5,143,854, 5,242,974, 5,252,743, 5,324,633, 5,384,261, 5,405,783, 5,412,087, 5,424,186, 5,445,934, 5,451,683, 5,482,867, 5,489,678, 5,491,074, 5,510,270, 5,527,681, 5,550,215, 5,571,639, 5,578,832, 5,593,839, 5,599,695, 5,624,711, 5,631,734, 5,677,195, 5,744,101, 5,744,305, 5,770,456, 5,795,716, 5,800,992, 5,831,070, 5,837,832, 5,856,101, 5,871,928, 5,858,659, 5,936,324, 5,968,740, 5,974,164, 5,981,185, 5,981,956, 6,025,601, 6,033,860, 6,040,138, and 6,090,555, which are all incorporated herein by reference in their entirety for all purposes.

Analogue when used in conjunction with a biomonomer or a biopolymer refers to natural and un-natural variants of the particular biomonomer or biopolymer. For example, a nucleotide analogue includes inosine and dideoxynucleotides. A nucleic acid analogue includes peptide nucleic acids. The foregoing is not intended to be exhaustive but rather representative. More information can be found in U.S. Patent 6156501.

Complementary or substantially complementary: Refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be substantially complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, substantial complementarity exists when an RNA or DNA strand will hybridize under selective hybridization conditions to its complement. Typically, selective hybridization will occur when there is at least about 65% complementarity over a stretch of at least 14 to 25 nucleotides, preferably at least about 75%, more preferably at least about 90% complementarity. See e. g., M. Kanehisa Nucleic Acids Res. 12:203 (1984), incorporated herein by reference.

Hybridization refers to the process in which two single-stranded polynucleotides bind non-covalently to form a stable double-stranded polynucleotide; triple-stranded hybridization is also theoretically possible. The resulting (usually) double-stranded polynucleotide is a "hybrid." The proportion of the population of polynucleotides that forms stable hybrids is referred to herein as the "degree of hybridization." Hybridizations are usually performed under stringent conditions, for example, at a salt concentration of no more than 1 M and a temperature of at least 25EC. For example, conditions of 5X SSPE (750 mM NaCl, 50 mM NaPhosphate, 5 mM EDTA, pH 7.4) and a temperature of 25-30°C are suitable for allele-specific probe hybridizations. For stringent conditions, see, for example, Sambrook, Fritsche and Maniatis. "Molecular Cloning A laboratory Manual" 2^{nd} Ed. Cold Spring Harbor Press (1989) which is hereby incorporated by reference in its entirety for all purposes above.

Nucleic acid refers to a polymeric form of nucleotides of any length, such as oligonucleotides or polynucleotides, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleoside sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be customized to stabilize or destabilize hybrid formation or enhance the specificity of hybridization with a complementary nucleic acid sequence as desired.

Oligonucleotide or polynucleotide is a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) or mimetics thereof which may be isolated from natural sources, recombinantly produced or artificially synthesized. A further example of a polynucleotide of the present invention may be a peptide nucleic acid (PNA). The invention also encompasses situations in which there is a non-traditional base pairing such as Hoogsteen base pairing which has been identified in certain tRNA molecules and postulated to exist in a triple helix. "Polynucleotide" and "oligonucleotide" are used interchangeably in this application.

Polymorphism refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms.

Primer is a single-stranded oligonucleotide capable of acting as a point of initiation for template-directed DNA synthesis under suitable conditions, e.g., buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 3 to 6 and up to 30 or 50 nucleotides. Short primer molecules generally require cooler temperatures to form sufficiently stable hybrid complexes with the template. A primer needs not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

Single Nucleotide Polymorphism or SNP occurs at a polymorphic site occupied by a single nucleotide, which is the site of variation between allelic sequences. This site of variation is usually both preceded by and followed by highly conserved sequences e.g., sequences that vary in less than 1/100 or 1/1000 members of the populations of the given allele. A SNP usually arises due to the substitution of one nucleotide for another at the polymorphic site. These substitutions include both transitions (i.e. the replacement of one purine by another purine or one pyrimidine by another pyrimidine) and transversions (i.e. the replacement of a purine by a pyrimidine or vice versa). SNPs can also arise from either a deletion of a nucleotide or from an insertion of a nucleotide relative to a reference allele.

Substrate refers to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations.

High density nucleic acid probe arrays, also referred to as "DNA Microarrays," have become a method of choice for monitoring the expression of a large number of genes.

A target molecule refers to a biological molecule of interest. The biological molecule of interest can be a ligand, receptor, peptide, nucleic acid (oligonucleotide or polynucleotide of RNA or DNA), or any other of the biological molecules listed in U.S. Patent No. 5,445,934 at col. 5, line 66 to col. 7, line 51. For example, if transcripts of genes are the interest of an experiment, the target molecules would be the transcripts. Other examples include protein fragments, small molecules, etc. Target nucleic acid refers to a nucleic acid (often derived from a biological sample) of interest. Frequently, a target molecule is detected using one or more probes. As used herein, a probe is a molecule for detecting a target molecule. It can be any of the molecules in the same classes as the target referred to above. A probe may refer to a nucleic acid, such as an oligonucleotide, capable of binding to a target nucleic acid of complementary sequence through one or more types of chemical bonds, usually through complementary base pairing, usually through hydrogen bond formation. As used herein, a probe may include natural (*i.e.* A, G, U, C, or T) or modified bases (7-deazaguanosine, inosine, etc.). In addition, the bases in probes may be joined by a linkage other than a phosphodiester bond, so long as the bond does not interfere with hybridization. Thus, probes may be peptide nucleic acids in which the constituent bases are joined by peptide bonds rather than phosphodiester linkages. Other examples of probes include antibodies used to detect peptides or other molecules, any ligands for detecting its binding partners. When referring to targets or probes as nucleic acids, it should be understood that there are illustrative embodiments that are not to limit the invention in any way.

In preferred embodiments, probes may be immobilized on substrates to create an array. An array may comprise a solid support with peptide or nucleic acid or other molecular probes attached to the support. Arrays typically comprise a plurality of different nucleic acids or peptide probes that are coupled to a surface of a substrate in different, known locations. These arrays, also described as "microarrays" or colloquially "chips" have been generally described in the art, for example, in Fodor et al., Science, 251:767-777 (1991), which is incorporated by reference for all purposes. Methods of forming high density arrays of oligonucleotides, peptides and other polymer sequences with a minimal number of synthetic steps are disclosed in, for example, 5,143,854, 5,252,743, 5,384,261, 5,405,783, 5,424,186, 5,429,807, 5,445,943, 5,510,270, 5,677,195, 5,571,639, 6,040,138, all incorporated herein by reference for all purposes. The oligonucleotide analogue array can be synthesized on a solid substrate by a variety of methods, including, but not limited to, light-directed chemical coupling, and mechanically directed coupling. See Pirrung et al., U.S. Patent No. 5,143,854 (see also PCT Application No. WO 90/15070) and Fodor et al., PCT Publication Nos. WO 92/10092 and WO 93/09668, U.S. Pat. Nos. 5,677,195, 5,800,992 and 6,156,501 which disclose methods of forming vast arrays of peptides, oligonucleotides and other molecules using, for example, light-directed synthesis techniques. See also, Fodor et al., Science, 251, 767-77 (1991). These procedures for synthesis of polymer arrays are now referred to as VLSIPS™ procedures. Using the VLSIPS™ approach, one heterogeneous array of polymers is converted, through simultaneous coupling at a number of reaction sites, into a different heterogeneous array. See, U.S. Patent Nos. 5,384,261 and 5,677,195.

Methods for making and using molecular probe arrays, particularly nucleic acid probe arrays are also disclosed in, for example, U.S. Patent Numbers 5,143,854, 5,242,974, 5,252,743, 5,324,633, 5,384,261, 5,405,783, 5,409,810, 5,412,087, 5,424,186, 5,429,807, 5,445,934, 5,451,683, 5,482,867, 5,489,678, 5,491,074, 5,510,270, 5,527,681, 5,527,681, 5,541,061, 5,550,215, 5,554,501, 5,556,752, 5,556,961, 5,571,639, 5,583,211, 5,593,839, 5,599,695, 5,607,832, 5,624,711, 5,677,195, 5,744,101, 5,744,305, 5,753,788, 5,770,456, 5,770,722, 5,831,070, 5,856,101, 5,885,837, 5,889,165, 5,919,523, 5,922,591, 5,925,517, 5,658,734, 6,022,963, 6,150,147, 6,147,205, 6,153,743, 6,140,044 and D430024, all of which are incorporated by reference in their entireties for all purposes.

Methods for signal detection and processing of intensity data are additionally disclosed in, for example, U.S. Patents Numbers 5,547,839, 5,578,832, 5,631,734, 5,800,992, 5,856,092, 5,936,324, 5,981,956, 6,025,601, 6,090,555, 6,141,096, 6,141,096, and 5,902,723. Methods for array based assays, computer software for data analysis and applications are additionally disclosed in, e.g., U.S. Patent Numbers 5,527,670, 5,527,676, 5,545,531, 5,622,829, 5,631,128, 5,639,423, 5,646,039, 5,650,268, 5,654,155, 5,674,742, 5,710,000, 5,733,729, 5,795,716, 5,814,450, 5,821,328, 5,824,477, 5,834,252, 5,834,758, 5,837,832, 5,843,655, 5,856,086, 5,856,104, 5,856,174, 5,858,659, 5,861,242, 5,869,244, 5,871,928, 5,874,219, 5,902,723, 5,925,525, 5,928,905, 5,935,793, 5,945,334, 5,959,098, 5,968,730, 5,968,740, 5,974,164, 5,981,174, 5,981,185, 5,985,651, 6,013,440, 6,013,449, 6,020,135, 6,027,880, 6,027,894, 6,033,850, 6,033,860, 6,037,124, 6,040,138, 6,040,193, 6,043,080, 6,045,996, 6,050,719, 6,066,454, 6,083,697, 6,114,116, 6,114,122, 6,121,048, 6,124,102, 6,130,046, 6,132,580, 6,132,996, 6,136,269 and attorney docket numbers 3298.1 and 3309, all of which are incorporated by reference in their entireties for all purposes.

Nucleic acid probe array technology, use of such arrays, analysis array based experiments, associated computer software, composition for making the array and practical applications of the nucleic acid arrays are also disclosed, for example, in the following U.S. Patent Applications: 07/838,607, 07/883,327, 07/978,940, 08/030,138, 08/082,937, 08/143,312, 08/327,522, 08/376,963, 08/440,742, 08/533,582, 08/643,822, 08/772,376, 09/013,596, 09/016,564, 09/019,882, 09/020,743, 09/030,028, 09/045,547, 09/060,922, 09/063,311, 09/076,575, 09/079,324, 09/086,285, 09/093,947, 09/097,675, 09/102,167, 09/102,986, 09/122,167, 09/122,169, 09/122,216, 09/122,304, 09/122,434, 09/126,645, 09/127,115, 09/132,368, 09/134,758, 09/138,958, 09/146,969, 09/148,210, 09/148,813, 09/170,847, 09/172,190, 09/174,364, 09/199,655, 09/203,677, 09/256,301, 09/285,658, 09/294,293, 09/318,775, 09/326,137, 09/326,374, 09/341,302, 09/354,935, 09/358,664, 09/373,984, 09/377,907, 09/383,986, 09/394,230, 09/396,196, 09/418,044, 09/418,946, 09/420,805, 09/428,350, 09/431,964, 09/445,734, 09/464,350, 09/475,209, 09/502,048, 09/510,643, 09/513,300, 09/516,388, 09/528,414, 09/535,142, 09/544,627, 09/620,780, 09/640,962, 09/641,081, 09/670,510, 09/685,011, and 09/693,204 and in the following Patent Cooperative Treaty (PCT) applications/publications: PCT/NL90/00081, PCT/GB91/00066, PCT/US91/08693, PCT/U591/09226, PCT/US91/09217, WO/93/10161, PCT/US92/10183, PCT/GB93/00147, PCT/US93/01152, WO/93/22680, PCT/US93/04145, PCT/US93/08015, PCT/US94/07106, PCT/US94/12305, PCT/GB95/00542, PCT/US95/07377, PCT/US95/02024, ACT/U596/05480, PCT/US96/11147, PCT/US96/14839, PCT/US96/15606, ACT/U597/01603, ACT/U597/02102, PCT/GB97/005566, ACT/U597/06535, PCT/GB97/01148, PCT/GB97/01258, PCT/US97/08319, PCT/US97/08446, PCT/US97/10365, PCT/US97/17002, PCT/US97/16738, PCT/US97/19665, PCT/US97/20313, ACT/US97/21209, PCT/US97/21782, PCT/US97/23360, ACT/U598/06414, ACT/US98/01206, PCT/GB98/00975, ACT/U598/04280, ACT/U598/04571, PCT/US98/05438, PCT/US98/05451, PCT/US98/12442, PCT/US98/12779, PCT/US98/12930, PCT/US98/13949, PCT/US98/15151, ACT/U598/15469, ACT/U598/15458, PCT/US98/15456, ACT/US98/16971, PCT/US98/16686, PCT/US99/19069, PCT/US98/18873, PCT/US98/18541, PCT/US98/19325, PCT/US98/22966, PCT/US98/26925, PCT/US98/27405 and PCT/IB99/00048, all of which are incorporated by reference in their entireties for all purposes. All the above cited patent applications and other references cited throughout this specification are incorporated herein by reference in their entireties for all purposes.

The embodiments of the invention will be described using GeneChip® high oligonucleotide density probe arrays (available from Affymetrix, Inc., Santa Clara, CA, USA) as exemplary embodiments. One of skill the art would appreciate that the embodiments of the invention are not limited to high density oligonucleotide probe arrays. In contrast, the embodiments of the invention are useful for analyzing any parallel large scale biological analysis, such as those using nucleic acid probe array, protein arrays, etc.

Gene expression monitoring using GeneChip® high density oligonucleotide probe arrays are described in, for example, Lockhart et al., 1996, Expression Monitoring By Hybridization to High Density Oligonucleotide Arrays, Nature Biotechnology 14:1675-1680; U.S. Patent Nos. 6,040,138 and 5,800,992, all incorporated herein by reference in their entireties for all purposes.

### II. GENE EXPRESSION USING UNIVERSAL ARRAYS

Expression monitoring of a large number of genes has been routinely achieved by hybridizing labeled sample mRNA, cDNA, or cRNA directly to spotted cDNA microarray and high-density oligonucleotide arrays that contain complementary sequences as probes. This requires pre-fabrication of arrays with desired probe sequences either by spotting known cDNA clones or de novo synthesis on solid surfaces of specific oligonucleotides based on known sequences of interested genes.

In one aspect of the invention, methods are provided to use a universal gene expression array with selected cipher probe sequences to monitor expression of any genes of known sequence without having to synthesize a new specific array or obtain the cDNA clones to spot a cDNA array.

A cipher probe is a nucleic acid probe that contain unique sequence. A cipher probe is also referred as a tag probe (referencing to its ability to bind to a tag sequence) or a capture probe. In preferred embodiments, the methods of the invention are useful for monitoring the expression of a large number of genes indirectly using a universal oligonucleotide array containing the unique sequence ciphers with mediator oligonucleotides. Therefore, generally, the cipher sequences on the array are pre-selected for not containing sequences identical or significantly similar to all known sequences or sequences of genes to be monitored for expression.

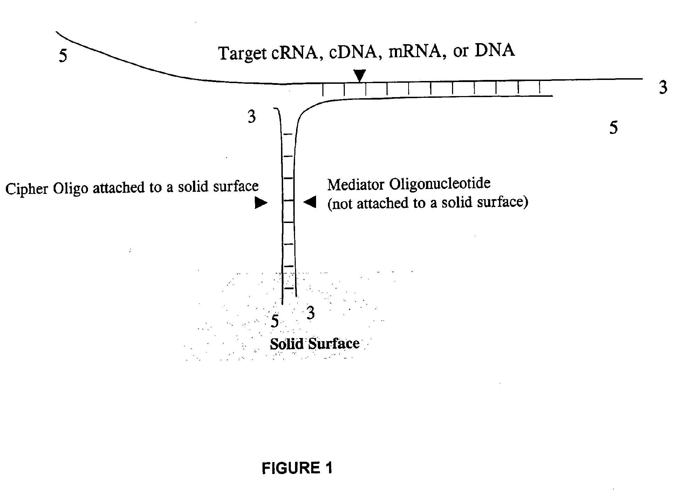

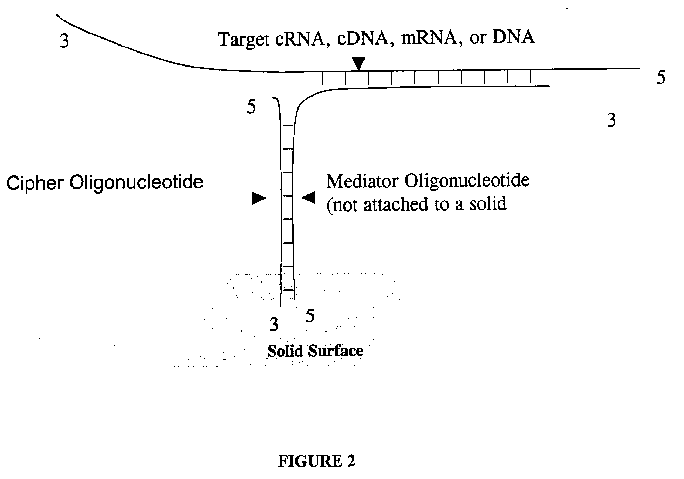

The mediators are nucleic acids that bind a cipher probe and a target nucleic acid. In some embodiments, the 3' portion of a mediator oligonucleotide is designed to hybridize to a selected region of the mRNA, cDNA, or cRNA, while its 5' portion is complementary to a specific cipher sequence on the universal array. The gene-specific hybridization of labeled nucleic acid samples to this array happen in the presence of the mediator oligonucleotides and target DNA/RNA, resulting in formation of a ternary complex between the target, mediator oligonucleotide, and cipher oligonucleotide attached to the solid surface (FIGURES 1 and 2). This approach enables monitoring mRNA expression of *any* genes of known sequence, including but not limited to alternatively spliced variants and variants at nucleotides level (e.g. SNP), without having to synthesize a new array or to obtain the cDNA clones to spot a cDNA array for the above purpose.

### III. SAMPLE PREPARATION

The methods of the invention are not limited to any particular method of sample preparation. A large number of well-known methods for isolating and purifying RNA are suitable for this invention.

One of skill in the art will appreciate that it is desirable to have nucleic samples containing target nucleic acid sequences that reflect the transcripts of interest. Therefore, suitable nucleic acid samples may contain transcripts of interest. Suitable nucleic acid samples, however, may also contain nucleic acids derived from the transcripts of interest. As used herein, a nucleic acid derived from a transcript refers to a nucleic acid for whose synthesis the mRNA transcript or a subsequence thereof has ultimately served as a template. Thus, a cDNA reverse transcribed from a transcript, an RNA transcribed from that cDNA, a DNA amplified from the cDNA, an RNA transcribed from the amplified DNA, etc., are all derived from the transcript and detection of such derived products is indicative of the presence and/or abundance of the original transcript in a sample. Thus, suitable samples include, but are not limited to, transcripts of the gene or genes, cDNA reverse transcribed from the transcript, cRNA transcribed from the cDNA, DNA amplified from the genes, RNA transcribed from amplified DNA, and the like. Transcripts, as used herein, may include, but not limited to pre-mRNA nascent transcript(s), transcript processing intermediates, mature mRNA(s) and degradation products. It is not necessary to monitor all types of transcripts to practice this invention. For example, one may choose to practice the invention to measure the mature mRNA levels only.

In one embodiment, such a sample is a homogenate of cells or tissues or other biological samples. Preferably, such sample is a total RNA preparation of a biological sample. More preferably in some embodiments, such a nucleic acid sample is the total mRNA isolated from a biological sample. Those of skill in the art will appreciate that the total mRNA prepared with most methods includes not only the mature mRNA, but also the RNA processing intermediates and nascent pre-mRNA transcripts. For example, total mRNA purified with poly (T) column contains RNA molecules with poly (A) tails. Those poly A+ RNA molecules could be mature mRNA, RNA processing intermediates, nascent transcripts or degradation intermediates.

Biological samples may be of any biological tissue or fluid or cells. Frequently the sample will be a "clinical sample" which is a sample derived from a patient. Clinical samples provide a rich source of information regarding the various states of genetic network or gene expression. Some embodiments of the invention are employed to detect mutations and to identify the function of mutations. Such embodiments have extensive applications in clinical diagnostics and clinical studies. Typical clinical samples include, but are not limited to, sputum, blood, blood cells (e.g., white cells), tissue or fine needle biopsy samples, urine, peritoneal fluid, and pleural fluid, or cells therefrom. Biological samples may also include sections of tissues such as frozen sections taken for histological purposes.

Another typical source of biological samples are cell cultures where gene expression states can be manipulated to explore the relationship among genes. In one aspect of the invention, methods are provided to generate biological samples reflecting a wide variety of states of the genetic network.

One of skill in the art would appreciate that it is desirable to inhibit or destroy RNase present in homogenates before homogenates can be used for hybridization. Methods of inhibiting or destroying nucleases are well known in the art. In some preferred embodiments, cells or tissues are homogenized in the presence of chaotropic agents to inhibit nuclease. In some other embodiments, RNase are inhibited or destroyed by heart treatment followed by proteinase treatment.

Methods of isolating total RNA and mRNA are also well known to those of skill in the art. For example, methods of isolation and purification of nucleic acids are described in detail in Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993) and Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993)).

In a preferred embodiment, the total RNA is isolated from a given sample using, for example, an acid guanidinium-phenol-chloroform extraction method and polyA⁺ mRNA is isolated by oligo (dT) column chromatography or by using (dT) magnetic beads (see, *e.g.,* Sambrook et al., Molecular Cloning: A Laboratory Manual (2nd ed.), Vols. 1-3, Cold Spring Harbor Laboratory, (1989), or Current Protocols in Molecular Biology, F. Ausubel *et al.*, ed. Greene Publishing and Wiley-Interscience, New York (1987)).

Most of eukaryotic mRNA have 3' poly (A) tails, some of eukaryotic and all of prokaryotic mRNA do not contain 3' poly (A) tails. It is often desirable to isolate mRNAs from RNA samples.

In one particularly preferred embodiment, total RNA is isolated from mammalian cells using RNeasy Total RNA isolation kit (QIAGEN). If mammalian tissue is used as the source of RNA, a commercial reagent such as TRIzol Reagent (GIBCOL Life Technologies). A second cleanup after the ethanol precipitation step in the TRIzol extraction using Rneasy total RNA isolation kit may be beneficial.

Hot phenol protocol described by Schmitt, et al., (1990) Nucleic Acid Res., 18:3091-3092 is useful for isolating total RNA for yeast cells.

Good quality mRNA may be obtained by, for example, first isolating total RNA and then isolating the mRNA from the total RNA using Oligotex mRNA kit (QIAGEN).

Total RNA from prokaryotes, such as E. coli. Cells, may be obtained by following the protocol for MasterPure complete DNA/RNA purification kit from Epicentre Technologies (Madison, WI).

Frequently, it is desirable to amplify the nucleic acid sample prior to hybridization. One of skill in the art will appreciate that whatever amplification method is used, if a quantitative result is desired, care must be taken to use a method that maintains or controls for the relative frequencies of the amplified nucleic acids to achieve quantitative amplification.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified nucleic acid.

Other suitable amplification methods include, but are not limited to polymerase chain reaction (PCR) (Innis, et al., PCR Protocols. A guide to Methods and Application. Academic Press, Inc. San Diego, (1990)), ligase chain reaction (LCR) (see Wu and Wallace, Genomics, 4: 560 (1989), Landegren, et al., Science, 241: 1077 (1988) and Barringer, et al., Gene, 89: 117 (1990), transcription amplification (Kwoh, et al., Proc. Natl. Acad. Sci. USA, 86: 1173 (1989)), and self-sustained sequence replication (Guatelli, et al., Proc. Nat. Acad. Sci. USA, 87: 1874 (1990)).

Cell lysates or tissue homogenates often contain a number of inhibitors of polymerase activity. Therefore, RT-PCR typically incorporates preliminary steps to isolate total RNA or mRNA for subsequent use as an amplification template. One tube mRNA capture method may be used to prepare poly(A)+ RNA samples suitable for immediate RT-PCR in the same tube (Boehringer Mannheim). The captured mRNA can be directly subjected to RT-PCR by adding a reverse transcription mix and, subsequently, a PCR mix.

In a particularly preferred embodiment, the sample mRNA is reverse transcribed with a reverse transcriptase and a primer consisting of oligo dT and a sequence encoding the phage T7 promoter to provide a single stranded DNA template. The second DNA strand is polymerized using a DNA polymerase with or without primers (See, U.S. Patent Application Serial Number: 09/102,167, and U.S. Provisional Application Serial No. 60/172,340, both incorporated herein by reference for all purposes). After synthesis of double-stranded cDNA, T7 RNA polymerase is added and RNA is transcribed from the cDNA template. Successive rounds of transcription from each single cDNA template results in amplified RNA. Methods of in vitro polymerization are well known to those of skill in the art (see, e.g., Sambrook, supra.) and this particular method is described in detail by Van Gelder, et al., Proc. Natl. Acad. Sci. USA, 87: 1663-1667 (1990). Moreover, Eberwine et al. Proc. Natl. Acad. Sci. USA, 89: 3010-3014 provide a protocol that uses two rounds of amplification via in vitro transcription to achieve greater than 10⁶ fold amplification of the original starting material thereby permitting expression monitoring even where biological samples are limited. In one preferred embodiment, the in-vitro transcription reaction may be coupled with labeling of the resulting cRNA with biotin using Bioarray high yield RNA transcript labeling kit (Enzo P/N 900182).

Before hybridization, the resulting cRNA may be fragmented. One preferred method for fragmentation employs Rnase free RNA fragmentation buffer (200 mM tris-acetate, pH 8.1, 500 mM potassium acetate, 150 mM magnesium acetate). Approximately 20 µg of cRNA is mixed with 8 µL of the fragmentation buffer. Rnase free water is added to make the volume to 40 µL. The mixture may be incubated at 94 °C for 35 minutes and chilled in ice.

It will be appreciated by one of skill in the art that the direct transcription method described above provides an antisense (aRNA) pool. Where antisense RNA is used as the target nucleic acid, the oligonucleotide probes provided in the array are chosen to be complementary to subsequences of the antisense nucleic acids. Conversely, where the target nucleic acid pool is a pool of sense nucleic acids, the oligonucleotide probes are selected to be complementary to subsequences of the sense nucleic acids. Finally, where the nucleic acid pool is double stranded, the probes may be of either sense as the target nucleic acids include both sense and antisense strands.

The protocols cited above include methods of generating pools of either sense or antisense nucleic acids. Indeed, one approach can be used to generate either sense or antisense nucleic acids as desired. For example, the cDNA can be directionally cloned into a vector (e.g., Stratagene's p Bluscript II KS (+) phagemid) such that it is flanked by the T3 and T7 promoters. In vitro transcription with the T3 polymerase will produce RNA of one sense (the sense depending on the orientation of the insert), while in vitro transcription with the T7 polymerase will produce RNA having the opposite sense. Other suitable cloning systems include phage lambda vectors designed for Cre-loxP plasmid subcloning (see e.g., Palazzolo et al., Gene, 88: 25-36 (1990)).

The biological sample should contain nucleic acids that reflects the level of at least some of the transcripts present in the cell, tissue or organ of the species of interest. In some embodiments, the biological sample may be prepared from cell, tissue or organs of a particular status. For example, a total RNA preparation from the pituitary of a dog when the dog is pregnant. In another example, samples may be prepared from E. Coli cells after the cells are treated with IPTG. Because certain genes may only be expressed under certain conditions, biological samples derived under various conditions may be needed to observe all transcripts. In some instance, the transcriptional annotation may be specific for a particular physiological, pharmacological or toxicological condition. For example, certain regions of a gene may only be transcribed under specific physiological conditions. Transcript annotation obtained using biological samples from the specific physiological conditions may not be applicable to other physiological conditions.

### IV. DESIGN OF UNIVERSAL ARRAYS AND MEDIATORS

### a) Array Designs

The preferred embodiments of the invention employ universal array that contain a large number cipher probes. Methods for designing and fabricating universal arrays are described in, for example, U.S. Patent Application Serial No. 09/536,841, which is incorporated herein by reference for all purposes.

In some embodiments, the cipher probes are at least 15, 20, 25, 30, 35, 40, 45 and 50 bases in length. In one probe selection method, all possible probes of given length is first generated. The probe sequences are compared with biological sequences in public and private databases. Probes that are complementary to known biological sequences are eliminated from the candidate probe pool. The remaining probes are selected for their hybridization characteristics. The selected cipher probes have similar hybridization characteristics and minimal homology to biological sequences. The hybridization characteristics may be selected based upon certain rules and/or based upon predicted hybridzation behaviour of the probes. Methods for selecting optimal probes for gene expression are disclosed in for example, U.S. Patent Nos. 5,800,992, and 6,040,138, U.S. Patent Application Serial No. 60/252,808, attorney docket number 3369, and U.S. Patent Application Serial No. 60/252,617, attorney docket number 3373, all incorporated here by reference for all purposes.

### b) Fabrication of Arrays

Methods for making and using molecular probe arrays, particularly nucleic acid probe arrays are also disclosed in, for example, U.S. Patent Numbers 5,143,854, 5,242,974, 5,252,743, 5,324,633, 5,384,261, 5,405,783, 5,409,810, 5,412,087, 5,424,186, 5,429,807, 5,445,934, 5,451,683, 5,482,867, 5,489,678, 5,491,074, 5,510,270, 5,527,681, 5,527,681, 5,541,061, 5,550,215, 5,554,501, 5,556,752, 5,556,961, 5,571,639, 5,583,211, 5,593,839, 5,599,695, 5,607,832, 5,624,711, 5,677,195, 5,744,101, 5,744,305, 5,753,788, 5,770,456, 5,770,722, 5,831,070, 5,856,101, 5,885,837, 5,889,165, 5,919,523, 5,922,591, 5,925,517, 5,658,734, 6,022,963, 6,150,147, 6,147,205, 6,153,743, 6,140,044 and D430024, all of which are incorporated by reference in their entireties for all purposes.

In brief, the light-directed combinatorial synthesis of oligonucleotide arrays on a glass surface proceeds using automated phosphoramidite chemistry and chip masking or optical direct write techniques. In one specific implementation, a glass surface is derivatized with a silane reagent containing a functional group, *e.g.*, a hydroxyl or amine group blocked by a photolabile protecting group. Photolysis through a photolithogaphic mask or micromirror arrays is used selectively to expose functional groups which are then ready to react with incoming 5'-photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences have been synthesized on the solid surface. Combinatorial synthesis of different oligonucleotide analogues at different locations on the array is determined by the pattern of illumination during synthesis and the order of addition of coupling reagents.

The universal array of the invention may be synthesized in 5'-3' direction (FIGURE 1) and 3'-5' direction (FIGURE 2). U.S. Patent Application Serial Number 09/490,580, which is incorporated herein by reference for all purposes, disclosed specific methods for synthesizing oligonucleotide probes on a substrate in 5'-3' direction.

In addition to photo-directed synthesis, other methods may also be employed for the fabrication of arrays with immobilized primers. For example, oligonucleotide synthesis may be conducted by selective delivery of reagents to specific locations using mechanic channels or ink-jet printers.

### c) Design of Mediators

One important aspect of the present invention is the use of mediator oligonucleotides which hybridize with both the cipher probes and a target nucleic acid (see, FIGURES 1 and 2). A mediator oligonucleotide should be at least 20, 25, 30, 35, 40, 45 or 50 base in length. Each mediator oligonucleotide should contain a region complementary to a cipher (tag probe) sequence. This region should be at least 10, 15, 20, 25 base long at its 3' portion (FIGURE 1) or 5' portion (FIGURE 2). Each mediator oligonucleotide should also contain a region complementary to a nucleic acid target. The region should be at least 10, 15, 20, 25 base long at 5' portion (FIGURE 1) or 3' (FIGURE 2) portion. Methods for selecting optimal probes for gene expression are disclosed in for example, U.S. Patent Nos. 5,800,992, and 6,040,138, U.S. Patent Application Serial No. 09/718,295, attorney docket number 3359, U.S. Patent Application, docket number 3359, and U.S. Patent Application Serial No. 09/745,965, attorney docket number 3373.1, all incorporated here by reference for all purposes.

The mediator oligonucleotides can be synthesized using commercially available DNA synthesizers such as the ABI 3948 Nucleic Acid Synthesis and Purification System (Applied Biosystems, Foster City, CA).

### V. HYBRIDIZATION AND WASHING

Nucleic acid hybridization simply involves contacting a probe and target nucleic acid under conditions where the probe and its complementary target can form stable hybrid duplexes through complementary base pairing.

It is generally recognized that nucleic acids are denatured by increasing the temperature or decreasing the salt concentration of the buffer containing the nucleic acids. Under low stringency conditions (e.g., low temperature and/or high salt) hybrid duplexes (e.g., DNA: DNA, RNA: RNA, or RNA: DNA) will form even where the annealed sequences are not perfectly complementary. Thus specificity of hybridization is reduced at lower stringency. Conversely, at higher stringency (e.g., higher temperature or lower salt) successful hybridization requires fewer mismatches.

One of skill in the art will appreciate that hybridization conditions may be selected to provide any degree of stringency. In a preferred embodiment, hybridization is performed at low stringency in this case in 6X SSPE-T at 37 C (0.005% Triton X-100) to ensure hybridization and then subsequent washes are performed at higher stringency (e.g., 1 X SSPE-T at 37 C) to eliminate mismatched hybrid duplexes. Successive washes may be performed at increasingly higher stringency (e.g., down to as low as 0.25 X SSPE-T at 37 C to 50 C) until a desired level of hybridization specificity is obtained. Stringency can also be increased by addition of agents such as formamide. Hybridization specificity may be evaluated by comparison of hybridization to the test probes with hybridization to the various controls that can be present (e.g., expression level control, normalization control, mismatch controls, etc.).

In general, there is a tradeoff between hybridization specificity (stringency) and signal intensity. Thus, in a preferred embodiment, the wash is performed at the highest stringency that produces consistent results and that provides a signal intensity greater than approximately 10% of the background intensity. Thus, in a preferred embodiment, the hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest.

Altering the thermal stability (Tₘ) of the duplex formed between the target and the probe using, e.g., known oligonucleotide analogues allows for optimization of duplex stability and mismatch discrimination. One useful aspect of altering the T m arises from the fact that adenine-thymine (A-T) duplexes have a lower Tₘ than guanine-cytosine (G-C) duplexes, due in part to the fact that the A-T duplexes have 2 hydrogen bonds per base-pair, while the G-C duplexes have 3 hydrogen bonds per base pair. In heterogeneous oligonucleotide arrays in which there is a non-uniform distribution of bases, it is not generally possible to optimize hybridization for each oligonucleotide probe simultaneously. Thus, in some embodiments, it is desirable to selectively destabilize G-C duplexes and/or to increase the stability of A-T duplexes. This can be accomplished, e.g., by substituting guanine residues in the probes of an array which form G-C duplexes with hypoxanthine, or by substituting adenine residues in probes which form A-T duplexes with 2,6 diaminopurine or by using the salt tetramethyl ammonium chloride (TMACl) in place of NaCl.

Methods of optimizing hybridization conditions are well known to those of skill in the art (see, e.g., Laboratory Techniques in Biochemistry and Molecular Biology, Vol. 24: Hybridization With Nucleic Acid Probes, P. Tijssen, ed. Elsevier, N.Y., (1993)).

### VI. SIGNAL DETECTION AND DATA ANALYSIS

In a preferred embodiment, the hybridized nucleic acids are detected by detecting one or more labels attached to the sample nucleic acids. The labels may be incorporated by any of a number of means well known to those of skill in the art. However, in a preferred embodiment, the label is simultaneously incorporated during the amplification step in the preparation of the sample nucleic acids. Thus, for example, polymerase chain reaction (PCR) with labeled primers or labeled nucleotides will provide a labeled amplification product. In a preferred embodiment, transcription amplification, as described above, using a labeled nucleotide (e.g. fluorescein-labeled UTP and/or CTP) incorporates a label into the transcribed nucleic acids. Alternatively, cDNAs synthesized using a RNA sample as a template, cRNAs are synthesized using the cDNAs as templates using in vitro transcription (IVT). A biotin label may be incorporated during the IVT reaction (Enzo Bioarray high yield labeling kit).

Alternatively, a label may be added directly to the original nucleic acid sample (e.g., mRNA, polyA mRNA, cDNA, etc.) or to the amplification product after the amplification is completed. Means of attaching labels to nucleic acids are well known to those of skill in the art and include, for example nick translation or end-labeling (e.g. with a labeled RNA) by kinasing of the nucleic acid and subsequent attachment (ligation) of a nucleic acid linker joining the sample nucleic acid to a label (e.g., a fluorophore).

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label. One particularly preferred method uses colloidal gold label that can be detected by measuring scattered light.

The label may be added to the target (sample) nucleic acid(s) prior to, or after the hybridization. So called "direct labels" are detectable labels that are directly attached to or incorporated into the target (sample) nucleic acid prior to hybridization. In contrast, so called "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an aviden-conjugated fluorophore will bind the biotin bearing hybrid duplexes providing a label that is easily detected. For a detailed review of methods of labeling nucleic acids and detecting labeled hybridized nucleic acids see Laboratory Techniques in Biochemistry and Molecular Biology, Vol. 24: Hybridization With Nucleic Acid Probes, P. Tijssen, ed. Elsevier, N.Y., (1993)).

Fluorescent labels are preferred and easily added during an in vitro transcription reaction. In a preferred embodiment, fluorescein labeled UTP and CTP are incorporated into the RNA produced in an in vitro transcription reaction as described above.

Means of detecting labeled target (sample) nucleic acids hybridized to the probes of the high density array are known to those of skill in the art. Thus, for example, where a colorimetric label is used, simple visualization of the label is sufficient. Where a radioactive labeled probe is used, detection of the radiation (e.g. with photographic film or a solid state detector) is sufficient.

In a preferred embodiment, however, the target nucleic acids are labeled with a fluorescent label and the localization of the label on the probe array is accomplished with fluorescent microscopy. The hybridized array is excited with a light source at the excitation wavelength of the particular fluorescent label and the resulting fluorescence at the emission wavelength is detected. In a particularly preferred embodiment, the excitation light source is a laser appropriate for the excitation of the fluorescent label.
The confocal microscope may be automated with a computer-controlled stage to automatically scan the entire high density array. Similarly, the microscope may be equipped with a phototransducer (e.g., a photomultiplier, a solid state array, a CCD camera, etc.) attached to an automated data acquisition system to automatically record the fluorescence signal produced by hybridization to each oligonucleotide probe on the array. Such automated systems are described at length in U.S. Patent No: 5,143,854, PCT Application 20 92/10092, and U.S. Application Ser. No. 08/195,889 filed on February 10, 1994. Use of laser illumination in conjunction with automated confocal microscopy for signal detection permits detection at a resolution of better than about 100 µm, more preferably better than about 50 µm, and most preferably better than about 25 µm.

One of skill in the art will appreciate that methods for evaluating the hybridization results vary with the nature of the specific probe nucleic acids used as well as the controls provided. In the simplest embodiment, simple quantification of the fluorescence intensity for each probe is determined. This is accomplished simply by measuring probe signal strength at each location (representing a different probe) on the high density array (*e.g.*, where the label is a fluorescent label, detection of the amount of florescence (intensity) produced by a fixed excitation illumination at each location on the array). Comparison of the absolute intensities of an array hybridized to nucleic acids from a "test" sample with intensities produced by a "control" sample provides a measure of the relative expression of the nucleic acids that hybridize to each of the probes.

One of skill in the art, however, will appreciate that hybridization signals will vary in strength with efficiency of hybridization, the amount of label on the sample nucleic acid and the amount of the particular nucleic acid in the sample. Typically nucleic acids present at very low levels (*e.g*., < 1pM) will show a very weak signal. At some low level of concentration, the signal becomes virtually indistinguishable from the background. In evaluating the hybridization data, a threshold intensity value may be selected below which a signal is not counted as being essentially indistinguishable from the background.

Suitable scanners, computer software for controlling the scanners and computer software for data management and analysis are available from commercial sources, such as Affymetrix, Inc., Santa Clara, CA.

The fluorescence intensity data (or other signals) detected may be processed as described for gene expression monitoring without extension reaction. Some of the data processing methods are described in, *e.g.,* U.S. Patent Nos. 6,040,138 and 5,800,992, U.S. Patent Application Serial Numbers 09/528,414, attorney docket numbers 3259.1, 3357.1, USSN unassigned, attorney docket number 3298.1, 60/189,558, attorney docket number 3309, 09/737,536, attorney docket number 3364, and 09/735,574, attorney docket number 3369.1, all incorporated herein in their entireties by reference for all purposes.

### VII. EXAMPLES

The following examples illustrate the methods of the invention using the GenFlex™ tag array (Affymetrix, Inc, Santa Clara, CA). The examples also demonstrate that the methods of the invention are capable of monitoring gene expression at high sensitivity and with good linearity.

GenFlex™ array contains about 2000 sets of 20-base long oligos that were selected not to be identical or significantly homologous to any published genomic sequences and have reasonable hybridization specificity and intensity. These are 20mers which were selected from all possible 20mers to have similar hybridization characteristics and minimal homology to sequences in the public databases. The Tag-probe set refers to the four sequences on the array used to analyze a given Tag, one of which (PM) is designed to the perfect complement of the Tag. Another probe is designed to be the mismatch-containing Tag probe with a different base at position 10 is referred to as "MM". Two additional control probes are included on the array, the "CPM" and the "CMM". These probes designed to be are the complements to the PM and MM probes, respectively.

An initial set of 20mer Tag-probe sequences was selected with closely matched melting temperatures. A further filter was applied to optimize and standardize the hybridization characteristics of the set. Finally, Tag-probe sequences were removed if they were identical or nearly identical to each other, to control sequences used on the array borders, or to sequences in the public databases at the time of the array design. Additional information about the GenFlex tag array is provided with application notes from Affymetrix' web site (http://www.affymetrix.com, last visited on December 17, 2000).

Eighteen mediator oligos were designed to monitor mRNA expression of 4 bacterial genes (BioB, BioC, BioD, and BioCre) and 4 human genes (GAPDH, b-actin, transferrin receptor, and an interferon inducible gene). Each mediator oligonucleotide contained a unique 20-base long sequence at its 5' portion complementary to a cipher (tag probe) sequence on the GenFlex array and its 3' portion was a 25-base long sequence same as selected region of the mRNA. The mRNA from HL-60 human myeloid cells was used to prepare the biotin-labeled cRNA using standard cRNA preparation protocol as described in GeneChip® *Expression Analysis Technical Manual.* The 18 mediator oligos were mixed with HL-60 cRNA and hybridized, washed, stained, and scanned in the same buffer and under same condition as recommended for standard GeneChip® expression arrays. The hybridization intensity is determined for each probe and plotted.

### a) Example 1

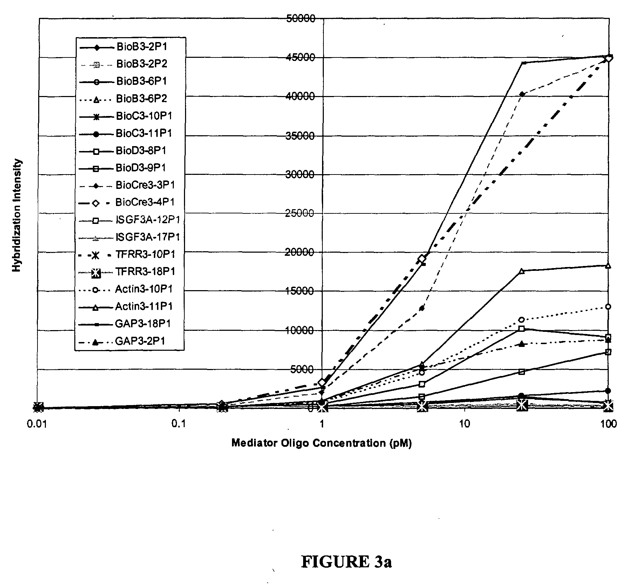

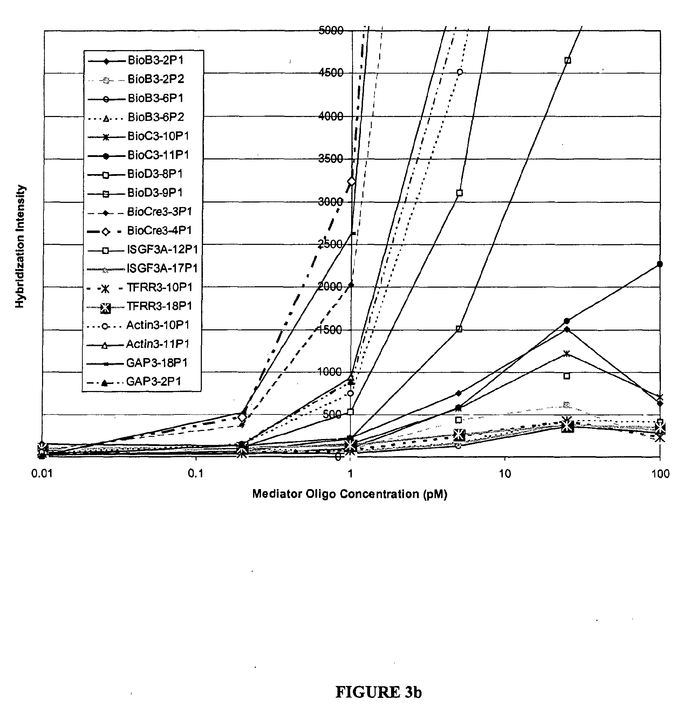

Four microgram of labeled HL-60 cRNA in 90 ul volume was hybridized together with labeled BioB, BioC, BioD, and BioCre cRNAs at the concentration of 1.5, 5, 25, 100 pM, respectively, to the GenFlex™ array with the 18 mediator oligos at various concentrations as indicated at X-axis. After hybridization, washing, staining, and scanning, the hybridization intensity to corresponding cipher oligos on GenFlex array was plotted against the concentration of the mediator oligos for each oligo (FIGURES 3a and 3b). The result shows that the hybridization intensity is dependent on the concentration of the mediator oligos. a) The data is plotted with the maximal value of 50,000 at the Y-axis. b) The data is plotted with the maximal value of 5,000 at the Y-axis.

### b) Example 2

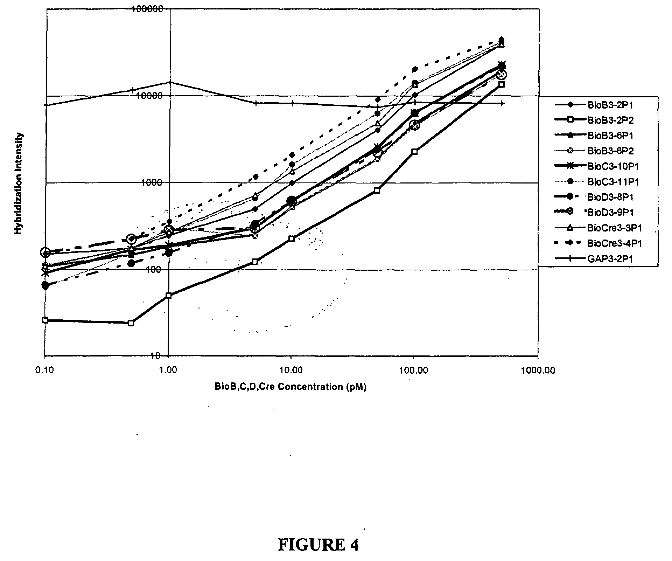

Equal amounts of BioB, BioC, BioD, and BioCre cRNAs at various concentrations as indicated was hybridized to the GenFlex array together with 4 microgram of HL-60 cRNA, in 90 ul volume in the presence of 50 pM of each 18 mediator oligos. After hybridization, washing, staining, and scanning, the hybridization intensity to corresponding cipher oligos was plotted against the concentration of the BioB, BioC, BioD, BioCre cRNAs for each oligo (FIGURE 4). The result shows that the hybridization intensity is linear to the concentration of the target cRNA between 0.1-500 pM range. It also shows that the sensitivity of the detection is at least 0.5pM for the target cRNA.

### CONCLUSION

The present inventions provide methods for analyzing a large number of RNAs. It is to be understood that the above description is intended to be illustrative and not restrictive. Many variations of the invention will be apparent to those of skill in the art upon reviewing the above description. By way of example, the invention has been described primarily with reference to the use of a high density oligonucleotide array, but it will be readily recognized by those of skill in the art that other nucleic acid arrays. The scope of the invention should be determined with reference to the appended claims, along with the full scope of equivalents to which such claims are entitled. All cited references, including patent and non-patent literature, are incorporated herewith by reference in their entireties for all purposes.

## Claims

1. A method for detecting a plurality of nucleic acid targets in a sample comprising:
hybridizing the sample with a plurality of mediator nucleic acids and a plurality of cipher probes immobilized on a substrate, wherein each of the mediator nucleic acids has a first subsequence that is complementary with one of the nucleic acid targets and a second subsequence that is complementary with one of the cipher probes; and
detecting the nucleic acid targets based upon the hybridization pattern.

2. The method of Claim 1 wherein the mediator nucleic acids and cipher probes are oligonucleotides.

3. The method of Claim 1 or 2 wherein the cipher probes do not substantially hybridize with the nucleic acid targets.

4. The method of Claim 4 wherein the cipher probes do not substantially hybridize with any nucleic acid in the sample.

5. The method of any one of claims 1 to 4 wherein the cipher probes are at least 15 bases in length.

6. The method of Claim 5 wherein the cipher probes are at least 20 bases in length.

7. The method of any one of claims 11 to 6 wherein the cipher probes are immobilized at density of at least 400 probes per cm².

8. The method of Claim 7 wherein the cipher probes are immobilized at a density of at least 1000 probes per cm².

9. The method of any one of claims 1 to 8 wherein the first subsequences of the mediator oligonucleotides are at least 15 bases in length.

10. The method of Claim 7 wherein the first subsequences are at least 20 bases in length.

11. The method of any one of claims 1 to 10 wherein the second subsequences are at least 15 bases in length.

12. The method of any one of claims 1 to 11 wherein the detecting comprises quantifying the binding of the nucleic acid targets to the cipher probes through the mediator probes.

13. The method of Claim 12 wherein the sample comprises a pool of mRNAs.

14. The method of Claim 12 wherein the sample comprises a pool of RNAs in vitro transcribed from a pool of cDNAs.

15. The method of any one of claims 1 to 14 wherein the pool of target nucleic acids is amplified from a biological sample by an in vivo or an in vitro method.

16. The method of any one of claims 1 to 14 wherein the pool of target nucleic acids comprises fluorescently labeled nucleic acids.

17. The method of any one of claims 1 to 16 wherein the cipher probes are synthesized in the 5'-3' direction on the substrate.

18. The method of Claim 17 wherein the cipher probes are synthesized using photo-directed synthesis.

19. The method of any one of claims 1 to 16 wherein the cipher probes are synthesized in the 3'-5' direction on the substrate.

20. The method of Claim 19 wherein the cipher probes are synthesized using photo-directed synthesis.

21. The method of any one of claims 1 to 20 wherein there are at least 3 mediator oligonucleotides and 3 corresponding cipher probes for each of the nucleic acid targets.

22. The method of Claim 21 wherein there are at least 5 mediator oligonucleotides and 5 corresponding cipher probes for each of the nucleic acid targets.

23. The method of Claim 22 wherein there are at least 10 mediator oligonucleotides and 10 corresponding cipher probes for each of the nucleic acid targets.

24. The method of Claim 23 wherein there are at least 20 mediator oligonucleotides and 20 corresponding cipher probes for each of the nucleic acid targets.
